(19) Europäisches Patentamt
European Patent Office
Office européen des brevets

(11) Publication number: **0 136 900**
**B1**

(12) **EUROPEAN PATENT SPECIFICATION**

(45) Date of publication of patent specification: **13.06.90**

(51) Int. Cl.$^5$: **A 61 L 31/00** // A61B19/08

(21) Application number: **84306663.0**

(22) Date of filing: **28.09.84**

(54) **Antimicrobial fabric for surgical drape.**

(30) Priority: **30.09.83 US 538062**

(43) Date of publication of application:
**10.04.85 Bulletin 85/15**

(45) Publication of the grant of the patent:
**13.06.90 Bulletin 90/24**

(84) Designated Contracting States:
**AT BE CH DE FR GB IT LI NL SE**

(56) References cited:
**EP-A-0 099 209**
**US-A-4 311 479**

**CHEMICAL ABSTRACTS, vol. 102, no. 18, 6th May 1985, page 76, abstract no. 150882c, Columbus, Ohio, US; & JP-A-59 150 174 (KOMATSU SEIREN CO. LTD.) 28-08-1984**

(73) Proprietor: **Surgikos, Inc.**
**One Johnson & Johnson Plaza**
**New Brunswick, N.J. 08903 (US)**

(72) Inventor: **Brown, Craig C.**
**4106 Woodcastle Court**
**Arlington Texas 76016 (US)**

(74) Representative: **Jones, Alan John et al**
**CARPMAELS & RANSFORD 43 Bloomsbury Square**
**London, WC1A 2RA (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the invention

This invention relates to nonwoven fabric which possesses antimicrobial properties and is useful in the manufacture of surgical drapes. The nonwoven fabric is treated with an antimicrobial agent which has activity against a broad spectrum of Gram negative and Gram positive bacteria and which will not become inactivated by contact with blood serum.

Background of the invention

Surgical drapes are used during surgical procedures to isolate the patient from the operating room personnel and the environment of the operating room. The surgical drapes were originally made of woven textile fabrics, but these fabrics have substantially been replaced by nonwoven fabrics. The advantages of the nonwoven fabrics are that they can be made repellent to retard or prevent liquid strike-through from the upper or the contaminated surface of the drape through the drape to the patient. The passage of liquid through a surgical drape is considered to be a source of bacterial contamination to the patient, possibly resulting in infection. In most disposable surgical drapes, the area immediately surrounding the operative site in the drape is reinforced with additional layers of nonwoven repellent fabric or with a combination of an absorbent fabric and an impervious plastic film. The use of an adsorbent fabric or other material such as an absorbent foam aroumd the operative site is advantageous because the surface of these materials helps to prevent fluid run off from the area surrounding the operative site into the operative wound site. Although the use of these absorbent materials offers an advantage, there is still a possibility that some fluid will flow into the wound site when the absorbent material has become saturated or if the fluid is present in greater quantity than the absorbent capacity of the absorbent material. This fluid could be contaminated with bacteria or may contact bacteria on the surface of the drape and become contaminated. Bacteria-contaminated fluid flowing into the wound site could cause post-operative infection problems for the surgical patient. For this reason surgical drapes which are treated with antimicrobial agents have been considered to be desirable.

Although there are large numbers of antimicrobial agents available to the manufacturer of surgical drapes, these antimicrobial agents have not been found to be suitable for this particular use for a number of reasons. The antimicrobial agent should be active against a broad spectrum of both Gram positive and Gram negative bacteria. Many of the antimicrobial agents are cationic in nature and react with the anionic surfactants that are commonly used in the binder systems to bind the fibers in the nonwoven fabrics. This inactivates the antimicrobial and renders it useless for the intended purpose. Other antimicrobial agents are not effective in contact with blood serum. Although the antimicrobial is effective in contact with other fluids, the presence of blood serum on the surface of the drape inactivates the antimicrobial and renders it useless. In addition, some antimicrobials are considered to be toxic to humans and, if they are capable of leaching from the nonwoven fabric in the presence of fluids found in the operating room, such as saline solutions, these antimicrobials are not considered to be desirable for use in surgical drapes. Other antimicrobials react with the binders or finishes in the fabric and form toxic adducts.

There have been prior attempts to incorporate antimicrobial agents into materials that will be useful for surgical drapes and similar products. U.S. Patent 3,310,459 suggests the formation of a latex impregnated, water-laid sheet for use as a surgical drape. An antimicrobial agent or bactericidal substance is suggested to be incorporated in the water used to form a cellulose web. The web is formed on normal paper-making equipment. A web formed in this manner does not have the properties desired as a fabric for use as a surgical drape.

U.S. Patent 3,521,625 suggests the use of a formaldehyde precursor in various absorbent products such as surgical dressings, disposable diapers and surgical drapes. When the formaldehyde precursor is contacted with water, formaldehyde is released which inhibits bacterial proliferation. Formaldehyde is not considered to be a desirable antimicrobial for contact with human skin.

US—A—4311479 discloses a method of indicating whether a fabric is impregnated with an antimicrobial compound. The fabric is impregnated with a cationic antimicrobial compound which has an anionic dye applied to a portion thereof so that it bonds ionically to the impregnant but not the fabric. On contact with a polar liquid, the dye or a dye-antimicrobial combination is released, serving to indicate that the fabric still possesses antimicrobial properties.

In addition, the particular antimicrobial agent used in the present invention, polyhexamethylene biguanide, has been suggested as an antimicrobial that has some utility in the treatment of textile materials. U.S. Patents 2,336,605; 2,990,425; 4,022,836; 2,863,919 disclose this utility.

A surgical drape is commercially available which contains an antimicrobial on the surface of the drape. The antimicrobial agent is a silicone quaternary ammonium salt. This surgical drape does not meet all of the desirable requirements for a product of this type because the antimicrobial is inactivated to a large degree in the presence of blood, which is commonly found on the surface of surgical drapes during surgical procedures. The efficacy of the antimicrobial agent used in this product is significantly diminished in the presence of blood.

Summary of the invention

The present invention provides a surgical drape containing antimicrobial agents which are not inactivated by blood serum and which have a very low saline extractability level and, therefore, have low toxicity to the patient. The surgical drape of the present invention is made with a cellulose-containing, nonwoven fabric which is bonded with a binding agent which contains little or no anionic surfactant or is made with a nonionic surfactant. The fabric contains a polyhexamethylene biguanide salt as the antimicrobial. In addition, the surgical drape may be treated with a rewetting agent which is an amphoteric surfactant to insure that the surface of the surgical drape has absorbent properties if absorbency is desired.

In addition to its use as an absorbent component in a surgical drape material, the antimicrobial-treated nonwoven fabric of the present invention can also be used for other applications in an operating room. It can be employed as an instrument wrap, a table cover and, if repellent treated, as a water-repellent drape material. When used in the present application, the term "surgical drape" is intended to apply to all such uses of the present antimicrobial-treated nonen fabric.

Detailed description of the invention

The present invention finds its greatest application in disposable surgical drapes. These drapes are made with a nonwoven fabric which is generally treated with a water-repellent agent. The treatment with a water-repellent agent provides resistance to liquid strike-through, particularly water, through the surface of the drape when the drape is in use. It is believed that the passage of liquid through the drape can carry bacteria from the upper surface of the drape into contact with the body of the patient and offers the possibility of introducing foreign bacteria into the patient, especially at the wound site of the surgical procedure. The surgical drapes are often constructed with a fenestration preformed in the drape. The fenestration is the opening through which the surgical procedure will be performed. There is generally a reinforcement around the fenestration to provide additional resistance to liquid penetration which might occur because of the presence of fluids in the area of the fenestration. The upper surface of the reinforcement area is generally made with an absorbent surface so that liquid will be absorbed on the surface to reduce the tendency of liquid to flow into the operative site. The lower surface of the reinforcement area is usually liquid-impervious film to prevent liquid strike-through to the operative site.

As previously indicated, attempts have been made to introduce antimicrobial agents into this absorbent material to lessen the possiblity of bacteria being transferred into the wound site. The selection of an antimicrobial that can be used for this purpose is complicated by the chemical nature of the binders that are used in the nonwoven fabrics wich form the drape. Many antimicrobials are cationic in nature and the binders used in the manufacture of nonwovens generally contain an anionic surfactant which is used as the emulsifying or dispersing surfactant in the manufacture of the polymeric binder. The cationic antimicrobial agents and the anionic surfactants are not only incompatible but often interreact to form adducts which may in themselves have toxic properties. It is, therefore, not possible to make a simple selection of any nonwoven fabric material and add one of the many available antimicrobials to the nonwoven fabric to form an effective bactericidal surface on the disposable drape.

The antimicrobial nonwoven fabric of the present invention is particularly useful as the fabric on the reinforcement area of a surgical drape. The nonwoven fabric may be made from any of the standard nonwoven fabric manufacturing processes. The nonwoven fabric used in the manufacture of surgical drapes generally weighs between 40 and 100 grams per square meter. The fabric generally contains some amount of cellulosic fiber, either in the form of regenerated cellulose, i.e., rayon, or woodpulp fibers. These fibers have absorbent properties which are desirable for the reasons given above. The nonwoven fabric may also contain synthetic fibers such as polyestter or polypropylene as is commonly found in nonwoven fabrics. The binder system that is used for these nonwoven fabrics must be one that contains little or no anionic surfactant in the formulation of binder or in the formulation of the polymer material on which the binder system is based. An excellent binder material that can be used for the purpose of the present invention is a copolymer of ethylene and vinyl acetate. This binder system is available as a dispersion in polyvinyl alcohol with no surfactant in the system. The binder is also available dispersed in water with a nonionic surfactant. Either form of the ethylene vinul acetate polymer is suitable in the practice of the present invention. The binder generally contains approximately 70% ethylene and 30% vinyl acetate and may contain small amounts of other ingredients such as crosslinking agents or other additives. Other binding systems that can be used are based on styrene butadiene polymers and acrylate polymers in which the anionic surfactant has been removed from the polymer.

The method of application of the binder to the formed web can be by any commercially available system. it is, however, preferred to incorporate all of the binder and the antimicrobial agent along with the rewetting agent into a mixture and spray this mixture onto a preformed web to give better control of the amount of antimicrobial in the finished product. The antimicrobial can also be applied by printing in a print pattern on one or both surfaces of the web. The binder is then cured by the usual curing techniques used in the manufacture of such nonwoven fabrics.

The antimicrobial agent that is used in the present invention.is a commercially available antimicrobial polyhexamethylene biguanide hydrochloride. The stoichiometric formulation for this material is

$$C_8H_{18}N_5CL(C_8H_{18}N_5CL)_n$$

where n is between 4.5 and 6.5. The structural formula is:

$$HCL\ NH_2(CH_2)_3[(CH_3)_2—NH—\overset{\overset{NH}{\|}}{C}—NH—\overset{\overset{NHCL}{\|}}{C}—NH(CH_2)_3]$$

This material is generally known as PHMB and is available from ICI North America as an aqueous liquid containing approximately 20% solids. It has antimicrobial activity against a broad spectrum of both Gram negative and Gram positive bacteria.

If it is desirable to provide absorbent characteristics to the nonwoven fabric, it is necessary to include a rewetting agent or rewetting surfactant in the formulation. This material is preferably an amphoteric surfactant so that it will not interfere with the antimicrobial agent. Suitable nonionic rewetting agents include betaine surfactants, nonylphenoxypoly (ethyleneoxy) ethane, polyethylene glycol esters and sucrose fatty acid esters. The preferred rewetting agent is cocamidopropyl betaine.

It is particularly important in the practice of the present invention to insure that the amount of antimicrobial agent on the surface of the nonwoven web is sufficient to give the desired bactericial activity but is not so great to pose a problem in the event that some of the antimicrobial is leached from the surface of the fabric when this fabric is wetted with an operating room liquid. The antimicrobial must be effectually tied into the surface of the web either by some attraction to the fibers in the web or to the binder itself. The amount of the antimicrobial that can be extracted from the surface of the web by leaching with a saline solution should be less than approximately 80 parts per million and preferably less than 50 ppm. Saline extraction levels of the antimicrobial greater than 80 ppm may cause problems of toxicity which must be avoided.

A typical formulation of binder, antimicrobial and rewetting agent would contain the following (amounts are in parts by weight):

| | Wet weight | Dry weight |
|---|---|---|
| Ethylene vinyl acetate | 157 | 86.35 |
| PHMB | 4.0 | 0.80 |
| Lexaine C | 4.8 | 1.44 |
| $NH_4CL$ (crosslinking agent) | 0.57 | 0.57 |
| Water | 302 | |
| Total | 468.57 | 89.16 |

The formulation is thoroughly mixed and is then applied to an unbonded, nonwoven web during the manufacturing process of the nonwoven fabric. The formulation can be sprayed at a level of approximately 15 to 30 grams per square meter of fabric, and the fabric is then dried to crosslink the binder. The formulation can be sprayed on both sides of the fabric, in which case the amount of formulation sprayed would be between 7.5 and 15 grams per square meter. The binder content of fabric is between 15 and 25 grams per square meter on a dry basis. The amount of PHMB in the formulatuon applied to the fabric is from 0.05 to 0.40 grams per square meter of fabric on a dry basis. The amount of antimicrobial agent in the binder formulation is approximately .25% to 2% based on weight. The presence of the antimicrobial in the fabric in amounts greater than 0.40 grams per square meter can result in a saline extraction level of greater than 80 parts per million, which is not desirable.

As indicated above, if it is desirable to maintain the absorbent properties of the fabric, a rewetting agent is included in the formulation. A standard test for absorption is to take a 12"×12" (30 cm×30 cm) piece of the fabric to be tested and apply it to a surface at a 45° angle. Forty (40) millileters of water are then dripped onto the surface of the fabric in a 40 second time period. If no liquid rolls down and off the fabric, the absorbency of the fabric is considered to be acceptable.

The determination of the extraction levels of the PHMB from the fabric by saline is performed in the following manner. A physiological saline solution (0.9% salt concentration) is heated to 40°C in an oven. Samples of the fabric to be treated are cut into 1" (2.5 cm) squares and placed in a jar with 15 milliliters of the heated solution for each 1 gram sample of fabric. The samples are allowed to wet evenly and held for five minutes with an occasional mixing. The liquid is then removed from the container and tested with the appropriate agent for the antimicrobial. The solutions for PHMB are 1 · N sodium hydroxide, cetyl trimethyl ammonium bromide, isopropyl alcohol and alkaline sodum hydrobromate. After the fabric has been extracted, the reagents are added to a flask which contains the extract and the absorbence at 428 nm against a blank solution by spectrophotometric determination is made. The amount of PHMB can be directly determined by comparing the absorbence of tested solution with that of a standard.

The bactericidal efficacy of the fabric is tested *in vitro* according to the following technique. Samples of the fabric to be tested are cut into 1" (2.5 cm) squares and placed in individual petri dishes. Suspensions of the test organisms are prepared using sheep's blood as a diluent with the test organism at a concentration of approximately $1 \times 10^5$ viable cells per ml. The inoculum at a rate of 0.2 ml is then added to the center of each sample square. The samples are placed in a high humidity chamber and removed after predetermined exposure times, as shown in the subsequent table. At the end of the exposure time, the samples are placed in a tube with a neutralizer and saturated. The tubes are then vortexed to insure removal of any surviving bacteria from the test sample. For each tube, 1 ml of neutralizer is placed in a petri dish and poured with molten TSA agar. These petri dishes are then incubated at 37°C for 48 hours, and the colony count is determined. In order to be an acceptable antimicrobial for the surface of the surgical drape, the $\log_{10}$ decrease in the bacteria should be at least 3 $\log s_{10}$. A 3 $\log_{10}$ decrease would indicate that 99.9% of the bacteria have been destroyed.

Example I

The following Table I shows the log kill against various organisms at 30 and 60 minutes for the material of the present invention as well as controls. The fabric was a nonwoven fabric containing rayon and woodpulp fibers. The binder containing the antimicrobial was sprayed on both sides of the fabric. The binder was a Hycar acrylate binder which contains an anionic surfactant or a 70—30 ethylene vinyl acetate copolymer dispersed with a nonionic surfactant. The bactericidal efficacy was tested by the method set forth above. Table I clearly indicates the bactericidal efficacy of the present invention and also shows that the use of a binder which contains an anionic surfactant cannot be used. Lexaine C is a cocamidopropyl betaine.

TABLE I

| | $\log_{10}$ decreases | | | |
|---|---|---|---|---|
| | P. aeruginosa | | S. aureus | |
| Sample | 30′ | 60′ | 30′ | 60′ |
| A<br>0.0% PHMB (control)<br>Hycar binder<br>0.17% Lexaine C surfactant | −0.136 | −0.000 | −0.023 | −0.035 |
| B<br>0.05% PHMB<br>Hycar binder<br>0.17% Lexaine C surfactant | −0.046 | −0.097 | −0.059 | +0.023 |
| C<br>0.0% PHMB (control)<br>EVA binder<br>0.24% Lexaine C surfactant | +0.021 | +0.041 | −0.017 | −0.017 |
| D<br>0.20% PHMB<br>EVA binder<br>0.24% Lexaine C surfactant | −4.342 | −4.342 | −3.102 | −4.579 |
| E<br>0.06% PHMB<br>EVA binder<br>0.24% Lexaine C surfactant | −2.000 | −3.301 | −2.568 | −3.091 |

Example II

Samples of a nonwoven fabric made with a mixture of rayon and woodpulp fibers were treated with a binder formulation containing PHMB as an antimicrobial. The binder formulation contained the following (on a dry basis):

| | |
|---|---|
| ethylene vinyl acetate copolymer | 86.35 |
| PHMB | 0.80 |
| Lexaine C | 1.44 |
| $NH_4Cl$ | 0.57 |

The formulation was applied as a 19% solution in water at a rate of approximately 20 grams per square meter of fabric. Three different samples of the fabric were tested for bactericidal activity against various bacteria, identified in Table II, using Test AATCC-100 in the presence of sheep's blood. A control which was the same fabric without the PHMB was also tested. These were tested against various organisms, as shown in Table II. The examination of data in Table II shows that the product of the present invention will kill over 99% of bacteria according to the test results.

TABLE II

| | # of surviving bacteria | | | Log decrease | | Percent kill | |
|---|---|---|---|---|---|---|---|
| | | | | S. aureus | | | |
| Sample | 0′ | 30′ | 60′ | 30′ | 60′ | 30′ | 60′ |
| 1 | 17,700 | 5 | 10 | −3.55 | −3.25 | 99.972 | 99.944 |
| 2 | 19,600 | 5 | 10 | −3.59 | −3.29 | 99.974 | 99.949 |
| 3 | 18,000 | 0 | 10 | −4.26 | −3.26 | 100.000 | 99.944 |
| Control | 18,800 | 17,200 | 17,700 | −0.03 | −0.02 | 8.511 | 5.851 |
| | | | | P. Aeruginosa | | | |
| 1 | 17,900 | 0 | 0 | −4.25 | −4.25 | 100.000 | 100.000 |
| 2 | 22,700 | 10 | 0 | −3.36 | −4.36 | 99.956 | 100.000 |
| 3 | 22,200 | 20 | 0 | −3.05 | −4.35 | 99.910 | 100.000 |
| Control | 26,600 | 19,800 | 27,400 | −0.12 | +0.02 | 25.564 | 0.000 |
| | | | | K. Pneumoniae | | | |
| 1 | 47,000 | 10 | 15 | −3.67 | −3.49 | 99.979 | 99.968 |
| 2 | 41,800 | 0 | 5 | −4.62 | −3.92 | 100.000 | 99.988 |
| 3 | 47,900 | 15 | 5 | −3.50 | −3.98 | 99.969 | 99.990 |
| Control | 44,800 | 38,500 | 51,400 | −0.06 | +0.06 | 14.063 | 0.000 |
| | | | | P. Vulgaris | | | |
| 1 | 38,300 | 9,500 | 100 | −0.60 | −2.58 | 75.196 | 99.739 |
| 2 | 31,500 | 5,300 | 10 | −0.78 | −3.50 | 83.175 | 99.968 |
| 3 | 38,000 | 9,900 | 220 | −0.58 | −2.24 | 73.947 | 99.421 |
| Control | 38,500 | 41,000 | 37,800 | +0.02 | −0.01 | 0.000 | 1.818 |

Example III

The test of Example II was also run comparing the product of the present invention as set forth in Example II with a commercial available antimicrobial drape. Those results are shown in Table III. In Table III, Sample A is the product of the present invention, and Sample B is the commercially available product using a silicone quaternary ammonium salt as the antimicrobial agent.

TABLE III

| Sample | # of surviving bacteria 0′ | 30′ | 60′ | Log decrease 30′ | 60′ | Percent kill 30′ | 60′ |
|---|---|---|---|---|---|---|---|
| | | | | S. aureus | | | |
| A | 19,700 | 45 | 5 | −2.64 | −3.59 | 99.772 | 99.975 |
| B | 17,100 | 17,900 | 10,200 | +0.02 | −0.22 | 0.000 | 40.351 |
| Control | 19,000 | 19,300 | 31,800 | +0.02 | +0.22 | 0.000 | 0.000 |
| | | | | P. Aeruginosa | | | |
| A | 23,200 | 25 | 0 | −2.97 | −4.37 | 99.892 | 100.000 |
| B | 21,600 | 28,500 | 33,200 | +0.12 | +0.19 | 0.000 | 0.000 |
| Control | 25,000 | 33,100 | 29,600 | +0.12 | +0.07 | 0.000 | 0.000 |
| | | | | K. Pneumoniae | | | |
| A | 39,000 | 5 | 0 | −3.89 | −4.59 | 99.987 | 100.000 |
| B | 36,400 | 42,500 | 69,400 | +0.07 | +0.21 | 0.000 | 0.000 |
| Control | 41,500 | 41,300 | 51,900 | 0.00 | +0.10 | 0.482 | 0.000 |
| | | | | P. Vulgaris | | | |
| A | 50,100 | 4,700 | 40 | −1.03 | −3.10 | 90.619 | 99.920 |
| B | 39,000 | 47,500 | 50,500 | +0.09 | +0.11 | 0.000 | 0.000 |
| Control | 40,000 | 37,300 | 53,600 | −0.03 | +0.13 | 6.750 | 0.000 |

Example IV

This example shows the use of PHMB in repellent-treated, nonwoven fabrics. Samples of unbonded webs were treated with a binder formulation which contained PHMB and a silicone-based repellent finish. The binder formulation contained the following ingredients in parts by weight:

| | Wet | Dry |
|---|---|---|
| Ethylene vinyl acetate copolymer | 450 | 225 |
| PHMB | 6.5 | 1.3 |
| Lexaine C | 4.8 | 1.4 |
| Silicone repellent finish | 4.6 | 4.6 |

The formulation was applied to a nonwoven fabric at the rate of 19.6 grams per square meter on a wet basis. The repellent add-on on a dry basis was 0.42 grams per square meter. The resultant fabric was tested for repellency and found to be water repellent. The fabric was tested for bactericidal activity against P. aeruginos and resulted in a 99.5% kill of the bacteria.

**Claims**

1. A surgical drape having antimicrobial properties comprising a nonwoven fabric bonded with a binder substantially free of anionic surfactants and containing from 0.05 to 0.40 grams per square meter of a polyhexamethylene biquanide hydrochloride as an antimicrobial agent, the saline extraction of the antimicrobial from the fabric by physiological saline being less than 80 parts per million.

2. The surgical drape of Claim 1 in which the nonwoven fabric was a weight of from 40 to 100 grams per square meter.

3. The surgical drape of Claim 1 or Claim 2 in which the binder is an ethylene vinyl acetate copolymer containing approximately 70% ethylene and 30% vinyl acetate.

4. The surgical drape of Claim 3 in which the binder is a dispersion in polyvinyl alcohol prior to being applied to the drape.

5. The drape of any one of Claims 1 to 4 in which the binder is applied to the fabric in an amount of from 15 to 25 grams per square meter.

6. The surgical drape of any one of Claims 1 to 5 containing a surfactant as a rewetting agent in an amount of from 0.25 to 0.50 grams per square meter.

7. The surgical drape of any one of Claims 1 to 6 in which the antimicrobial is present in an amount of 0.15 grams per square meter.

8. A method of preparing a nonwoven fabric having antimicrobial properties and suitable for use as a surgical drape comprising applying to a preformed web of unbonded fibers an aqueous binder formulation containing:

a) from 95% to 98% by weight of an ethylene vinyl acetate copolymer binder substantially free of anionic surfactant;

b) from 0.25% to 2% by weight of a polyhexamethylene biguanide hydrochloride,

said binder being applied to said web in an amount of from 15 to 30 grams per square meter of fabric.

9. The method of Claim 8 in which the binder also contains from 1% to 2% by weight of cocamidopropyl betaine.

**Patentansprüche**

1. Chirurgisches Tuch mit mikrobiziden Eigenschaften, welches Tuch einen Faservliesstoff umfaßt, der mit einem Bindemittel gebunden ist, welches im wesentlichen von anionischen grenzflächenaktiven Mitteln frei ist, und der 0,05 bis 0,40 g/m$^2$ an Polyhexamethylenbiguanidhydrochlorid als ein mikrobizides Mittel enthält, wobei die Extraktion des mikrobiziden Mittels durch Salzlösungen aus dem Stoff durch physiologische Kochsalzlösung weniger als 80 TpM beträgt.

2. Chirurgisches Tuch nach Anspruch 1, worin das Faservliesstoff ein Flächengewicht von 40 bis 100 g/m$^2$ hat.

3. Chirurgisches Tuch nach Anspruch 1 oder Anspruch 2, worin das Bindemittel ein Ethylen-Vinylacetat-Copolymer mit einem Gehalt von etwa 70% Ethylen und 30% Vinylacetat ist.

4. Chirurgisches Tuch nach Anspruch 3, wobei das Bindemittel, bevor es auf das Tuch aufgetragen worden ist, als eine Dispersion in Polyvinylalkohol vorgelegen ist.

5. Tuch nach einem der Ansprüche 1 bis 4, wobei das Bindemittel auf den Stoff in einer Menge von 15 bis 25 g/m$^2$ aufgetragen worden ist.

6. Chirurgisches Tuch nach einem der Ansprüche 1 bis 5, welches ein grenzflächenaktives Mittels als Wiederbenetzungsmittel in einer Menge von 0,25 bis 0,50 g/m$^2$ enthält.

7. Chirurgisches Tuch nach einem der Ansprüche 1 bis 6, worin das mikrobizide Mittel in einer Menge von 0,15 g/m$^2$ vorliegt.

8. Verfahren zur Herstellung eines Faservliesstoffes mit mikrobiziden Eigenschaften, der für eine Verwendung als chirurgisches Tuch geeignet ist, welches Verfahren das Auftragen auf eine vorgeformte Bahn aus ungebundenen Fasern, von einer wässerigen Bindemittelformulierung umfaßt, welche enthält:

a) 95 Gew.-% bis 98 Gew.-% eines Bindemittels aus Ethylen-Vinylacetat-Copolymer, welches im wesentlichen von anionischem grenzflächenaktivem Mittel frei ist;

b) 0,25 Gew.-% bis 2 Gew.-% an einem Polyhexamethylenbiguanidhydrochlorid,

wobei dieses Bindemittel auf die Bahn in einer Menge von 15 bis 30 g/m$^2$ Stoff angetragen wird.

9. Verfahren nach Anspruch 8, wobei das Bindemittel auch 1 Gew.-% bis 2 Gew.-% Cocamidopropylbetain enthält.

**Revendications**

1. Drap chirurgical possédant des propriétés antimicrobiennes 'qui comprend' une étoffe non tissée liée à l'aide d'un liant sensiblement exempt de surfactifs anioniques et contenant de 0,05 à 0,40 g/m$^2$ de chlorhydrate de polyhexaméthylène-biguanide à titre d'agent antibactérien, l'extraction saline de l'agent antimicrobien de l'étoffe par la matière saline physiologique étant inférieure à 80 ppm.

2. Drap chirurgical selon la revendication 1, dans lequel le poids de l'étoffe non tissée est de 40 à 100 g/m$^2$.

3. Drap chirurgical selon la revendication 1 ou 2, dans lequel le liant est un copolymère éthylène/acétate de vinyl contenant environ 70% d'éthylène et 30% d'acétate de vinyle.

4. Drap chirurgical selon la revendication 3, dans lequel le liant est une dispersion dans l'alcool polyvinylique avant son application au drap.

5. Drap selon l'une quelconque des revendications 1 à 4, dans lequel le liant este appliqué à l'étoffe à raison de 15 à 25 g/m$^2$.

6. Drap chirurgical selon l'une quelconque des revendications 1 à 5, qui contient un surfactif en qualité d'agent remouillant à raison de 0,25 à 0,50 g/m$^2$.

7. Drap chirurgical selon l'une quelconque des revendications 1 à 6, dans lequel l'agent antimicrobien est présent à raison de 0,15 g/m$^2$.

8. Procédé de préparation d'une étoffe non tissée possédant des propriétés antimicrobiennes et convenant comme drap chirurgical qui consiste à appliquer à un tissu préformé de fibres non liées une formulation aqueuse de liant contenant:

a) de 95 à 98% en poids d'un copolymère liant éthylène/acétate de vinyle sensiblement exempt de surfactif anionique;

b) de 0,25 à 2% en poids d'un chlorhydrate de polyhexaméthylène biguanide,

ledit liant étant appliqué audit tissu à raison de 15 à 30 g/m² d'étoffe.

9. Procédé selon la revendication 8, dans lequel le liant contient également de 1 à 2% en poids de cocamidopropyl-bétaïne.